# EUROPEAN PATENT SPECIFICATION

(11) **EP 4 006 169 B1**
(45) Date of publication and mention of the grant of the patent: **23.10.2024**
(21) Application number: 20923230.5
(22) Date of filing: 13.05.2020
(51) Int. Cl.: C12Q 1/6806, C12Q 1/70, C12Q 1/6851, C12R 1/93

(54) **PRETREATMENT METHOD, PRETREATMENT SOLUTION, KIT FOR VIRUS NUCLEIC ACID DETECTION, AND USE THEREOF**
VORBEHANDLUNGSVERFAHREN, VORBEHANDLUNGSLÖSUNG, KIT ZUR DETEKTION VON VIRUSNUKLEINSÄUREN UND VERWENDUNG DAVON
PROCÉDÉ DE PRÉTRAITEMENT, SOLUTION DE PRÉTRAITEMENT, KIT POUR LA DÉTECTION D'ACIDES NUCLÉIQUES VIRAUX ET LEUR UTILISATION

(30) Priority: 04.03.2020 CN 202010143226
(43) Date of publication of application: 01.06.2022
(73) Proprietor: Sansure Biotech Inc., Changsha, Hunan 410205 (CN)
(72) Inventor: DAI, Lizhong, Changsha, Hunan 410205 (CN); JI, Bozhi, Changsha, Hunan 410205 (CN); DENG, Zhongping, Changsha, Hunan 410205 (CN); LIU, Jia, Changsha, Hunan 410205 (CN); TAN, Deyong, Changsha, Hunan 410205 (CN); FAN, Xu, Changsha, Hunan 410205 (CN)
(74) Representative: Novagraaf Technologies
(86) International application number: PCT/CN2020/090052
(87) International publication number: WO 2021/174673

(56) References cited:
- WO-A1-2005/042030
- WO-A1-2009/153299
- WO-A2-01/01129
- AU-A1- 2006 214 059
- CN-A- 1 706 959
- CN-A- 1 940 087
- CN-A- 101 970 660
- CN-A- 103 184 297
- CN-A- 104 131 006
- CN-A- 105 392 363
- CN-A- 110 982 876
- US-A1- 2012 238 017
- US-A1- 2016 106 854
- CROMEANS THERESA; JOTHIKUMAR NARAYANAN; LEE JEONGSU; COLLINS NIKAIL; BURNS CARA C.; HILL VINCENT R.; VINJÉ JAN: "A new solid matrix for preservation of viral nucleic acid from clinical specimens at ambient temperature", JOURNAL OF VIROLOGICAL METHODS, ELSEVIER BV, NL, vol. 274, 11 September 2019 (2019-09-11), NL, XP085893955, ISSN: 0166-0934, DOI: 10.1016/j.jviromet.2019.113732

## Description

### TECHNICAL FIELD

The present invention relates to the field of detecting a nucleic acid of a virus sample, and particularly relates to a virus sample pretreatment method and a pretreatment solution for pretreating a DNA/RNA virus.

### BACKGROUND

Currently, there are mainly two types of conventional virus pretreatment solutions: a Hank's matrix-based pretreatment solution and a guanidine salt-based pretreatment solution. The Hank's solution is a common balanced salt solution (BSS) for virus transport. Due to various factors, the Hank's solution can preserve a respiratory virus (such as an influenza virus, a novel coronavirus, etc.) for only a few hours. After a few hours, the morphology of the virus can be affected, which can affect the efficiency of detecting a viral nucleic acid. After an extended time, bacteria and fungi can easily grow, which lowers the pH value of the Hank's solution and accelerates degradation of the virus. Therefore, it is difficult to use the Hank's solution for long-term preservation of the virus and detection of a viral nucleic acid. The concentration of guanidine hydrochloride or Rnasin in the guanidine salt-based virus pretreatment solution is relatively high (3 M to 5 M). A high concentration of a guanidine salt is suitable for virus inactivation at room temperature. However, the high concentration of the guanidine salt greatly inhibits nucleic acid extraction or purification (such as based on magnetic beads), the implementation of which requires multiple times of washing or the use of a spin column.

The extraction free nucleic acid release and amplification technology (EFNART), simply referred to as the "one-step" technique, refers to directly performing a nucleic acid amplification test for a sample by directly combining a sample nucleic acid releasing agent with a strong alkaline property and a highly compatible amplification system in a circumstance where no nucleic acid extraction or purification of the sample is required. Operations performed based on the "one-step method" will greatly reduce time for detecting a viral nucleic acid, particularly for an RNA virus. The present invention is expected to save time by 60% or more and improve detection efficiency by 50% in comparison with a traditional amplification method performed after nucleic acid extraction.

In the event of a major epidemic (for example, the 2019 novel coronavirus (2019-nCoV) epidemic that occurred from 2019 to 2020), detection based on the one-step method is preferred because it saves detection time, improves efficiency, and will greatly contribute to controlling the epidemic.

However, existing common virus pretreatment solutions, such as the Hank's pretreatment solution and the guanidine salt-based pretreatment solution, cannot be well used for subsequent detection based on the one-step method. Thus, it is difficult to apply the existing common virus pretreatment solutions to scenarios in which rapid detection and screening of virus samples are required.

Therefore, there is a need in the art for a sample pretreatment solution that can be adequate for use in detection of a viral nucleic acid based on the one-step method. CN104131006A is an example of a method and a kit for extracting adenovirus DNA from sputum samples. The buffer comprises 50 mM Tris-HCl at pH 8.0, 100 mM EDTA at pH 8.0, 100 mM NaCl, 1% SDS, and 0.5mg/ml proteinase K. The present invention's pretreatment solution has increased amplification efficiency compared to the buffer in CN104131006A.

### SUMMARY

In view of this, in a first aspect, the present invention provides a pretreatment method of detecting a nucleic acid of a virus, the method comprising: mixing a sample stored in a pretreatment solution with a nucleic acid releasing agent and a qPCR amplification reagent, wherein the pretreatment solution comprises: Tris-HCl, EDTA-2Na, sodium chloride, a ribonuclease (RNase) inhibitor, and an antibiotic;
wherein the Tris-HCl is present at a concentration of about 10 mM to about 200 mM, preferably at a concentration of about 80 mM to about 120 mM, and most preferably at a concentration of about 100 mM; the EDTA-2Na is present at a concentration of about 8 mM to about 50 mM, preferably at a concentration of about 10 mM to about 15 mM, and most preferably at a concentration of about 10 mM; the sodium chloride is present at a concentration of about 0.5% (w/v) to about 2% (w/v), preferably at a concentration of about 0.8% (w/v) to about 1% (w/v), and most preferably at a concentration of about 0.9% (w/v); the "ribonuclease (RNase) inhibitor" refers to a RNase protein inhibitor (RNasin) present at a concentration of about 2 U/mL to about 800 U/mL, for example, about 40 U/mL, about 50 U/mL, and about 100 U/mL, preferably at a concentration of about 10 U/mL to about 30 U/mL; and most preferably at a concentration of 20 U/mL; and
wherein the pH value ranges from 6.5 to 8.0, preferably from 7.0 to 8.0, and is most preferably 7.5.

The antibiotic comprises, but is not limited to, Proclin antibiotics (such as Proclin 300 and Proclin 950) and NaN₃.

For example, in a case where Proclin 300 is used as the antibiotic, the concentration of Proclin 300 may be about 0.01% (v/v). For another example, in a case where Proclin 950 is used as the antibiotic, the concentration of Proclin 950 may be about 0.04% (v/v), but the present invention is not limited thereto.

In a specific embodiment, the pretreatment solution comprises Tris-HCl at a concentration of 100 mM, EDTA-2Na at a concentration of 10 mM, sodium chloride at a concentration of 0.9% (w/v), the RNase inhibitor at a concentration of 20 U/mL, and Proclin 950 at a concentration of 0.04% (v/v).

The aforementioned pretreatment solution is adjusted to a pH value of 7.5.

As used herein, the term "sample" refers to a sample that may contain a virus. The sample may be derived from human or animal blood, feces, urine, oral epithelial cells, exfoliated cells, buccal swabs, throat swabs, etc.

The term "pretreatment" mentioned in the present invention refers to treatment prior to performing a test for a sample, particularly treatment prior to detection of a nucleic acid of the sample (performed based on the one-step method).

The "pretreatment solution" mentioned in the present invention refers to a liquid for pretreating a virus sample.

In the present invention, the virus may be a DNA virus or an RNA virus.

In a preferred embodiment, the virus is an RNA virus.

In a more preferred embodiment, the virus is a coronavirus (such as the 2019-nCoV), a respiratory syncytial virus, and an enterovirus.

A nucleic acid detection reaction solution prepared by the pretreatment method of the present invention can be used to directly perform qPCR, without requiring an extraction or purification process, thereby improving detection efficiency and reducing detection time. The antibiotic and the RNase inhibitor employed in the present invention can be used for pretreatment of a DNA virus or an RNA virus, and can prevent the activity of a preserved virus from being affected by various microorganisms, such as bacteria, growing at room temperature. The RNA virus is more easily degraded due to the ubiquitous RNase. Damage to RNA that is caused by samples (various sample types such as oral epithelial cells, exfoliated cells, throat swabs, etc.) or sampling consumables in a medical sampling process is avoided while the method of the present invention is used, which is very conducive to long-term preservation and detection of the RNA virus.

It should be noted that in a case where the sample treated with the pretreatment solution of the present invention is not stored but directly (i.e., 0 hr after treatment) subjected to EFNART-based detection, as confirmed in the examples below, components of the pretreatment solution of the present invention have an effect of enhancing RT-PCT.

In a second aspect, the present invention provides a method of detecting a viral nucleic acid in a sample, comprising: directly performing detection by using the reaction solution prepared by the aforementioned method to perform qPCR amplification.

A sample releasing agent refers to a chemical agent that can release a nucleic acid in a sample, for example, a chemical agent with strong acidity or strong basicity. An exemplary sample releasing agent may be one or several of components comprising 0.01-0.5 mmol/L surfactin, 100-200 mmol/L potassium chloride, 50-200 mmol/L lithium chloride, triethanolamine dodecyl sulfate with a mass/volume ratio of 0.1-1%, ethyl phenyl polyethylene glycol (NP-40) with a volume/volume ratio of 0.1-1%, sodium dodecyl sulfonate with a mass/volume ratio of 0.01-2%, ethanol with a volume/volume ratio of 0.05-1%, etc., but the present invention is not limited thereto.

A qPCR amplification reagent refers to a reagent for a real-time quantitative nucleic acid amplification test. It can be understood by those skilled in the art that the qPCR amplification reagent usually contains DNA polymerase, dNTP, a PCR buffer solution, etc. For example, when RNA detection is to be performed, reverse transcriptase may also be further comprised. It is not difficult to understand that those skilled in the art can determine the components and concentration of a PCR reaction reagent according to specific needs (for example, the type and the content of a virus, etc.).

The term "one-step" mentioned in the present invention refers to the extraction free nucleic acid release and amplification technology (EFNART). The EFNART refers to directly performing a nucleic acid amplification test for a sample by directly combining a sample nucleic acid releasing agent with strong basicity and a highly compatible amplification system in a circumstance where no nucleic acid extraction or purification of the sample is required.

When detection of a viral nucleic acid in the sample is performed based on the one-step method, the sample in the preservation solution or pretreatment solution, the sample releasing agent, and the qPCR reaction solution are directly amplified after being mixed. Mixing may be performed at a general ratio in the art. In an exemplary embodiment, the sample in the preservation solution or the pretreatment solution, the sample releasing agent, and the qPCR reaction solution may be present in the ratio of about 5:5:40, about 10:10:30, or about 5:15:30 (v/v), but the present invention is not limited thereto.

In a third aspect, the present invention provides a pretreatment solution for detecting a nucleic acid of a virus, comprising: Tris-HCl, EDTA-2Na, sodium chloride, an RNase inhibitor, and an antibiotic,
wherein the Tris-HCl is present at a concentration of about 10 mM to about 200 mM, preferably at a concentration of about 80 mM to about 120 mM, and most preferably at a concentration of about 100 mM; the EDTA-2Na is present at a concentration of about 8 mM to about 50 mM, preferably at a concentration of about 10 mM to about 15 mM, and most preferably at a concentration of about 10 mM; the sodium chloride is present at a concentration of about 0.5% (w/v) to about 2% (w/v), preferably at a concentration of about 0.8% (w/v) to about 1% (w/v), and most preferably at a concentration of about 0.9% (w/v); the RNase inhibitor as defined above is present at a concentration of about 2 U/mL to about 800 U/mL, preferably at a concentration of about 10 U/mL to about 30 U/mL, and most preferably at a concentration of 20 U/mL; and
wherein the pH value ranges from 6.5 to 8.0, preferably from 7.0 to 8.0, and is most preferably 7.5.

The antibiotic comprises, but is not limited to, Proclin antibiotics (such as Proclin 300 and Proclin 950) and NaN₃.

For example, in a case where Proclin 300 is used as the antibiotic, the concentration of Proclin 300 may be about 0.01% (v/v). For another example, in a case where Proclin 950 is used as the antibiotic, the concentration of Proclin 950 may be about 0.04% (v/v), but the present invention is not limited thereto.

In a specific embodiment, the pretreatment solution comprises Tris-HCl at a concentration of 100 mM, EDTA-2Na at a concentration of 10 mM, sodium chloride at a concentration of 0.9% (w/v), the RNase inhibitor at a concentration of 20 U/mL, and Proclin 950 at a concentration of 0.04% (v/v).

The aforementioned pretreatment solution is adjusted to a pH value of 7.5.

In some specific embodiments, the virus may be a DNA virus or an RNA virus.

In a preferred embodiment, the virus is an RNA virus.

In a more preferred embodiment, the virus is a coronavirus (such as the 2019-nCoV), a respiratory syncytial virus, and an enterovirus.

In a fourth aspect, the present invention provides use of a pretreatment solution in preparation of a kit for detecting a virus by employing nucleic acid amplification based on the one-step method.

In a fifth aspect, the present invention provides a kit for detecting a viral nucleic acid based on the one-step method, the kit comprising the aforementioned pretreatment solution.

Further, the kit further comprises a sample releasing agent and a qPCR amplification reagent.

### BRIEF DESCRIPTION OF THE DRAWINGS

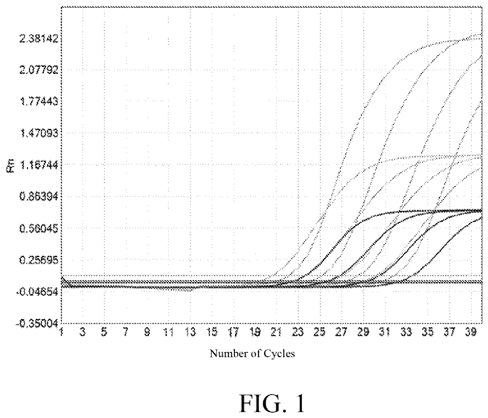

FIG. 1 is a graph of the results of "One Step" testing of the nucleic acid of the 2019-nCoV samples gradient-diluted by using the pretreatment method of the present invention and after being preserved at room temperature for 72 hr.

### DETAILED DESCRIPTION

The present invention will be described in detail below in conjunction with specific embodiments and examples, and the advantages and various effects of the present invention will be more clearly presented therefrom. It should be understood by those skilled in the art that these specific embodiments and examples are used to illustrate the present invention, but not to limit the present invention.

### Example 1. Pretreatment and rapid detection of a respiratory syncytial virus (RSV) in an oral swab sample performed in the Present Invention

In order to evaluate the virus pretreatment solution in the present invention, comparative analysis was performed on the virus pretreatment solution of the present invention (Tris-HCl at a concentration of 100 mM, EDTA-2Na at a concentration of 10 mM, sodium chloride at a concentration of 0.9% (w/v), RNasin at a concentration of 20 U/mL, and Proclin 950 at a concentration of 0.04% (v/v)), a saline solution, and commercially available virus pretreatment solutions. A comparison method was employed by performing dilution (1:9, v/v) pretreatment on an RSV throat swab sample that was clinically diagnosed to be positive. Direct amplification of the sample was performed at 0 hr, 24 hr, 48 hr, and 72 hr of the pretreatment time respectively under a pretreatment condition of a room temperature of 25°C. The detection efficiency of real-time quantitative PCR (real-time qPCR) under the room temperature pretreatment condition was compared by Ct values to evaluate the effects of different pretreatment solutions on virus pretreatment. A qPCR amplification test was employed by using the EFNART "one-step" technique, such that a real-time qPCR amplification test was directly performed in a PCR amplification tube at a ratio of a pretreatment solution with the sample: a nucleic acid releasing agent: a PCR amplification reagent of 10:10:30.

Real-time qPCR amplification testing procedures are as shown in Table 1, and the results are as shown in Table 2.

**Table 1**

| Step | Temperature | Time | Number of Cycles |
|---|---|---|---|
| Reverse transcription | 60°C | 30 min | 1 |
| Pre-denaturation | 95°C | 1 min | 1 |
| Denaturation | 95°C | 15 sec | 40-45 |
| Annealing, extension, and fluorescence collection | 60°C | 30 sec | |

**Table 2**

| | Ct Value by Detection in Original Sample | | | | Ct Value by Detection in 10-fold Diluted Sample | | | |
|---|---|---|---|---|---|---|---|---|
| Pretreatment Time | 0 hr | 24 hr | 48 hr | 72 hr | 0 hr | 24 hr | 48 hr | 72 hr |
| Pretreatment solution of the present invention | 30.69 | 31.05 | 30.98 | 31.24 | 33.56 | 33.98 | 33.78 | 34.01 |
| Saline solution | 31.04 | 32.15 | 33.24 | 34.65 | 34.12 | 35.26 | 36.42 | 36.98 |
| Hank's pretreatment solution | 35.12 | 38.20 | No Ct | No Ct | No Ct | No Ct | No Ct | No Ct |
| Guanidine hydrochloride pretreatment solution | No Ct | No Ct | No Ct | No Ct | No Ct | No Ct | No Ct | No Ct |

The aforementioned results showed that pretreatment was performed with the virus pretreatment method in the present invention at room temperature for 24 hr, and the pretreatment method can adequately pretreat the gradient-diluted RSV sample. In addition, testing could be directly performed by means of EFNART. After pretreatment was performed for a long time based on a saline solution matrix, the Ct values could be delayed, and the nucleic acid of the virus was degraded to a certain extent, which affected the amplification efficiency. In contrast, the Hank's pretreatment solution and the guanidine salt pretreatment solution, which are commonly used and commercially available, cannot provide an effective way of performing virus pretreatment and a PCR amplification test in this scheme.

### Example 2. Nucleic acid pretreatment and rapid detection after purification of 2019-nCoV sample performed in the present invention

In order to evaluate the virus pretreatment solution in the present invention, comparative analysis was performed on the virus pretreatment solution of the present invention (Tris-HCl at a concentration of 100 mM, EDTA-2Na at a concentration of 10 mM, sodium chloride at a concentration of 0.9% (w/v), RNasin at a concentration of 20 U/mL, and Proclin 300 at a concentration of 0.01% (v/v)), a saline solution, and commercially available virus pretreatment solutions. A comparison method was employed by performing dilution (1:9, v/v) pretreatment on a 2019-nCoV nucleic acid that was clinically diagnosed to be positive. Direct amplification of the sample was performed at 0 hr, 24 hr, 48 hr, and 72 hr of the pretreatment time respectively under the pretreatment condition of a room temperature of 25°C. The testing efficiency of real-time qPCR under the room temperature pretreatment condition was compared by Ct values to evaluate the effects of different pretreatment solutions on virus pretreatment. A qPCR amplification test was employed by using the EFNART "one-step" technique, such that a real-time qPCR amplification test was directly performed in a PCR amplification tube at a ratio of a pretreatment solution with the nucleic acid: a nucleic acid releasing agent: a PCR amplification reagent of 10: 10:30. The results are as shown in Table 3. The 2019-nCoV nucleic acid gradient-diluted by using the virus pretreatment method in the present invention was stored at room temperature for 72 hr. The results of the "One Step" testing are as shown in FIG. 1.

**Table 3**

| | Ct Value by Detection in Original Sample | | | | Ct Value by Detection in 10-fold Diluted Sample | | | |
|---|---|---|---|---|---|---|---|---|
| Pretreatment Time | 0 hr | 24 hr | 48 hr | 72 hr | 0 hr | 24 hr | 48 hr | 72 hr |
| Pretreatment solution of the present invention | 28.59 | 28.68 | 29.01 | 28.97 | 32.01 | 31.89 | 32.14 | 32.20 |
| Saline solution | 28.95 | 29.48 | 29.98 | 30.54 | 33.01 | 3.21 | 33.14 | 34.28 |
| Hank's pretreatment solution | 31.02 | 32.35 | 33.62 | No Ct | No Ct | No Ct | No Ct | No Ct |
| Guanidine hydrochloride pretreatment solution | No Ct | No Ct | No Ct | No Ct | No Ct | No Ct | No Ct | No Ct |

The aforementioned results showed that pretreatment was performed with the virus pretreatment method in the present invention at room temperature for 24 hr, and the pretreatment method can adequately pretreat the gradient-diluted 2019-nCoV nucleic acid. In addition, testing could be directly performed by means of EFNART. After pretreatment was performed for a long time based on a saline solution matrix, the Ct values could be delayed, and the nucleic acid of the virus was degraded to a certain extent, which affected the amplification efficiency. In contrast, the Hank's pretreatment solution and the guanidine salt pretreatment solution, which are commonly used and commercially available, cannot provide an effective way of performing virus pretreatment and a PCR amplification test in this scheme.

### Example 3. Pretreatment and rapid detection of Enterovirus general type (EV) throat swab sample performed in the present invention

In order to evaluate the virus pretreatment solution in the present invention, comparative analysis was performed on the virus pretreatment solution of the present invention (Tris-HCl at a concentraion of 100 mM, EDTA-2Na at a concentraion of 10 mM, sodium chloride at a concentraion of 0.9% (w/v), SDS at a concentraion of 0.1%, and Proclin 950 at a concentraion of 0.04% (v/v)), a saline solution, and commercially available virus pretreatment solutions. A comparison method was employed by performing dilution (1:9, v/v) pretreatment on an EV general type throat swab sample that was clinically diagnosed to be positive. Direct amplification of the sample was performed at 0 hr, 24 hr, 48 hr, and 72 hr of the pretreatment time respectively under the pretreatment condition of a room temperature of 25°C. The detection efficiency of real-time qPCR under the room temperature pretreatment condition was compared by Ct values to evaluate the effects of different pretreatment solutions on virus pretreatment. A qPCR amplification test was employed by using the EFNART "one-step" technique, such that a real-time qPCR amplification test was directly performed in a PCR amplification tube at a ratio of a pretreatment solution with the sample: a nucleic acid releasing agent: a PCR amplification reagent of 10:10:30. The results are as shown in Table 4:

**Table 4**

| | Ct Value by Detection in Original Sample | | | | Ct Value by Detection in 10-fold Diluted Sample | | | |
|---|---|---|---|---|---|---|---|---|
| Pretreatment Time | 0 hr | 24 hr | 48 hr | 72 hr | 0 hr | 24 hr | 48 hr | 72 hr |
| Pretreatment solution of the present invention | 26.12 | 26.32 | 26.25 | 26.34 | 29.65 | 29.78 | 29.82 | 29.16 |
| Saline solution | 27.32 | 27.15 | 28.64 | 29.01 | 30.98 | 31.65 | 32.27 | 32.59 |
| Hank's pretreatment solution | 30.12 | 30.54 | 33.28 | 33.42 | No Ct | No Ct | No Ct | No Ct |
| Guanidine hydrochloride pretreatment solution | No Ct | No Ct | No Ct | No Ct | No Ct | No Ct | No Ct | No Ct |

The aforementioned results showed that pretreatment was performed with the virus pretreatment method in the present invention at room temperature for 24 hr, and the pretreatment method can adequately pretreat the gradient-diluted EV sample. In addition, testing could be directly performed by means of EFNART. After pretreatment was performed for a long time based on a saline solution matrix, the Ct values could be delayed, and the nucleic acid of the virus was degraded to a certain extent, which affected the amplification efficiency. In contrast, the Hank's pretreatment solution and the guanidine salt pretreatment solution, which are commonly used and commercially available, cannot provide an effective way of performing virus pretreatment and a PCR amplification test in this scheme.

### Example 4. Pretreatment capacity of virus pretreatment solution of the present Invention for RNA virus

Compared with DNA viruses, pretreatment and detection of RNA viruses are more susceptible to environmental factors due to higher requirements. A pretreatment process involving the RNase contained in consumables has a particularly vital impact on testing of the RNA virus. In order to evaluate the pretreatment effect of the virus pretreatment solution in the present invention (tTris-HCl at a concentraion of 100 mM, EDTA-2Na at a concentraion of 10 mM, sodium chloride at a concentraion of 0.9% (w/v), Rnasin at a concentraion of 20 U/mL, and Proclin 950 at a concentraion of 0.04% (v/v)) on the RNA virus, the nucleic acid extracted from a 2019-nCoV sample pretreated with the virus pretreatment solution of the present invention was divided into two parts (A/B). At the same time, a nucleic acid (C) extracted from a 2019-nCoV sample with a same concentration that was pretreated in the saline solution was prepared. In addition, 0.25 µg/mL RNase A was respectively added into solution A and solution C. The three solutions containing the 2019-nCoV nucleic acid were pretreated at room temperature (25°C) for 24 hr. Then the RNA virus was pretreated and tested with the pretreatment solution in the present invention by adopting the EFNART "one-step" technique. Detection was conducted by directly performing a real-time qPCR amplification test in a PCR amplification tube at a ratio of a pretreatment solution with the nucleic acid: a nucleic acid releasing agent: a PCR amplification reagent of 10:10:30. The results are as shown in Table 5.

**Table 5**

| 2019-nCoV Nucleic Acid Pretreatment Condition | Ct Value by Detection | | |
|---|---|---|---|
| Pretreated nucleic acid sample A (pretreatment solution of the present invention containing 0.25 g/mL Rnase A) | FAM 30.8 | HEX 26.8 | ROX 29.6 |
| Pretreated nucleic acid sample B (pretreatment solution of the present invention not containing 0.25 g/mL Rnase A) | FAM 30.9 | HEX 27.1 | ROX 30.2 |
| Pretreated nucleic acid sample C (pretreatment with saline solution containing 0.25 g/mL Rnase A) | FAM No Ct | HEX 27.1 | ROX No Ct |
| Pretreated nucleic acid sample D (pretreatment with saline solution not containing 0.25 g/mL Rnase A) | FAM 32.2 | HEX 29.4 | ROX 32.9 |

It was proved by the experiments that in a case where pretreated nucleic acid sample D was taken as the reference, addition of 0.25 µg/mL RNase A in the present invention did not affect the effect of detecting a nucleic acid of 2019-nCoV, and effective components in the present invention could digest the RNase, thus reducing the effect of the RNase on the experimental testing, so that the efficiency of direct detection of the RNA virus could be guaranteed. Judging from pretreated nucleic acid sample C, addition of the 0.25 µg/mL RNase A included in the experimental conditions could digest and degrade the RNA in an experiment, thereby greatly affecting direct detection of the RNA virus and leading to risk of misses.

### Example 5. Pretreatment and rapid detection of hepatitis B virus (HBV) serum sample by using virus pretreatment solution of the present invention

In order to evaluate the effect of the virus pretreatment solution in the present invention on DNA virus preservation and amplification testing, comparative analysis was performed on the virus pretreatment solution of the present invention (Tris-HCl at a concentration of 100 mM, EDTA-2Na at a concentration of 10 mM, sodium chloride at a concentration of 0.9% (w/v), RNasin at a concentration of 20 U/mL, and Proclin 950 at a concentration of 0.04% (v/v)), a saline solution, and commercially available virus pretreatment solutions. A comparison method was employed by performing dilution (1:9, v/v) pretreatment on an HBV serum sample that was clinically diagnosed to be positive. Direct amplification of the sample was performed at 0 hr, 24 hr, 48 hr, and 72 hr of the pretreatment time respectively under the pretreatment condition of a room temperature of 25°C. The testing efficiency of real-time qPCR under the room temperature pretreatment condition was compared by Ct values to evaluate the effects of different pretreatment solutions on virus pretreatment. A qPCR amplification test was employed by using the EFNART "one-step" technique, such that a real-time qPCR amplification test was directly performed in a PCR amplification tube at a ratio of a pretreatment solution with the DNA virus: a nucleic acid releasing agent: a PCR amplification reagent of 10:10:30. The results are as shown in Table 6.

**Table 6**

| | Ct Value by Detection in HBV Serum Sample | | | |
|---|---|---|---|---|
| Pretreatment Time | 0 hr | 24 hr | 48 hr | 72 hr |
| Pretreatment solution of the present invention | 32.23 | 31.98 | 32.24 | 32.67 |
| Saline solution | 33.43 | 33.18 | 33.54 | 33.87 |
| Hank's pretreatment solution | 33.74 | 33.49 | 33.85 | 34.18 |
| Guanidine hydrochloride pretreatment solution | No Ct | No Ct | No Ct | No Ct |

The aforementioned results showed that although the original design of the virus pretreatment method in the present invention was aimed at virus matrix preservation for RNA viruses for performing amplification based on the "one-step method", the virus pretreatment method can also be used for preservation and detection of DNA viruses by performing amplification based on the one-step method.

### Example 6. Verification of effectiveness of various components of virus pretreatment solution of the present invention

In order to verify the effectiveness of various components in the present invention, after relevant components in the present invention were adjusted and reduced, the sample was preserved and was subjected to pretreatment and a comparison experiment. Separately optimized components included an RNase inhibitor, an antibiotic, EDTA-2Na, etc. Concentrations of the adjusted various components are as shown in Table 7. qPCR amplification testing was directly performed after RSV was preserved with the pretreatment solutions at room temperature for 24 hr that were prepared at different concentrations, and the detection efficiency of real-time qPCR under the room temperature pretreatment condition was compared by Ct values to evaluate the effects of different pretreatment solutions on virus pretreatment. A qPCR amplification test was employed by using the EFNART "one-step" technique, such that a real-time qPCR amplification test was directly performed in a PCR amplification tube at a ratio of a pretreatment solution with the sample: a nucleic acid releasing agent: a PCR amplification reagent of 10: 10:30.

**Table 7**

| Detection Effects of Separately Optimized components in the Present Invention on Pretreatment | | | | | | | |
|---|---|---|---|---|---|---|---|
| RNase inhibitor | | Antibiotic (Proclin 950) | | EDTA-2Na | | Solution pH Value Adjustment | |
| Concentration | Ct value | Concentration | Ct value | Concentration | Ct value | pH value | Ct value |
| 0 | No Ct | 0 | No Ct | 0 | 38.2 | 4 | 38.54 |
| 2 U/mL | 31.54 | 0.01% | 31.48 | 10 mM | 31.4 | 6.5 | 31.64 |
| 40 U/mL | 32.4 | 0.04% | 32.4 | 50 mM | 33.4 | 8 | 31.81 |
| 8000 U/mL | 35.42 | 1% | 37.62 | 200 mM | No Ct | 12 | No Ct |

Based on the aforementioned experiments, the chemical components in the present invention were necessary components, and performed best in the pretreatment and detection of the RNA virus by employing one-step RT-PCR at the concentrations in the present invention.

## Claims

1. A pretreatment method of detecting a nucleic acid of a virus, the method comprising:
mixing a sample stored in a pretreatment solution with a nucleic acid releasing agent and a qPCR amplification reagent, wherein the pretreatment solution comprises Tris-HCl, EDTA-2Na, sodium chloride, a ribonuclease (RNase) inhibitor, and an antibiotic;
wherein the concentration of Tris-HCl is 10 mM to 200 mM, the concentration of EDTA-2Na is 8 mM to 50 mM, the concentration of sodium chloride is 0.5% (w/v) to 2% (w/v), the concentration of the RNase inhibitor is 2 U/mL to 800 U/mL, and the concentration of the antibiotic is 0.005% to 0.05% in the pretreatment solution; and
wherein the pretreatment solution has a pH of 6.5-8.0.

2. The method according to claim 1, wherein the RNAse inhibitor is RNAsin and/or the antibiotic is chosen from the group consisting of Proclin antibiotics, preferably Proclin 950 or Proclin 300, and NaN₃.

3. The method according to claim 1, wherein the concentration of Tris-HCl is 80 mM to 120 mM, the concentration of EDTA-2Na is 10 mM to 15 mM, the concentration of sodium chloride is 0.8% (w/v) to 1% (w/v), and the concentration of the RNase inhibitor is 10 U/mL to 30 U/mL in the pretreatment solution.

4. The method according to any one of claims 1-3, wherein the virus is a DNA virus or an RNA virus, preferably an RNA virus, and more preferably a coronavirus, such as 2019-nCoV.

5. A pretreatment solution for detecting a nucleic acid of a virus, the pretreatment solution comprising: Tris-HCl, EDTA-2Na, sodium chloride, a ribonuclease (RNase) inhibitor, and an antibiotic,
wherein the concentration of Tris-HCl is 10 mM to 200 mM, the concentration of EDTA-2Na is 8 mM to 50 mM, the concentration of sodium chloride is 0.5% (w/v) to 2% (w/v), the concentration of the RNase inhibitor is 2 U/mL to 800 U/mL, and the concentration of the antibiotic is 0.005% to 0.05%; and
wherein the pretreatment solution has a pH of 6.5-8.0.

6. The pretreatment solution according to claim 5, wherein the RNAse inhibitor is RNAsin and/or the antibiotic is chosen from the group consisting of Proclin antibiotics, preferably Proclin 950 or Proclin 300, and NaN₃.

7. The pretreatment solution according to claim 5, wherein the concentration of Tris-HCl is 80 mM to 120 mM, the concentration of EDTA-2Na is 10 mM to 15 mM, the concentration of sodium chloride is 0.8% (w/v) to 1% (w/v), and the concentration of the RNase inhibitor is 10 U/mL to 30 U/mL.

8. The pretreatment solution according to 5-7, wherein the virus is a DNA virus or an RNA virus, preferably an RNA virus, and more preferably a coronavirus, such as 2019-nCoV.

9. Use of the pretreatment solution according to any one of claims 5-7 in preparation of a kit for detecting a virus by employing nucleic acid amplification based on the one-step method.

10. A kit for detecting a nucleic acid of a virus based on the one-step method, the kit comprising the pretreatment solution defined according to any one of claims 5-7.

11. The kit according to claim 10, wherein the kit further comprises a nucleic acid releasing agent and a qPCR amplification reagent.

## Patentansprüche

1. Vorbehandlungsverfahren zum Nachweisen einer Nukleinsäure eines Virus, das Verfahren umfassend: Mischen einer Probe, die in einer Vorbehandlungslösung aufbewahrt wird, mit einem Nukleinsäurefreisetzungsmittel und einem qPCR-Amplifikationsreagens, wobei die Vorbehandlungslösung Tris-HCl, EDTA-2Na, Natriumchlorid, einen Ribonuklease-Inhibitor (RNase-Inhibitor) und ein Antibiotikum umfasst;
wobei die Konzentration von Tris-HCl 10 mM bis 200 mM beträgt, die Konzentration von EDTA-2Na 8 mM bis 50 mM beträgt, die Konzentration von Natriumchlorid 0,5 % (w/v) bis 2 % (w/v) beträgt, die Konzentration des RNase-Inhibitors 2 U/ml bis 800 U/ml beträgt und die Konzentration des Antibiotikums 0,005 % bis 0,05 % in der Vorbehandlungslösung beträgt; und
wobei die Vorbehandlungslösung einen pH-Wert von 6,5-8,0 aufweist.

2. Verfahren nach Anspruch 1, wobei der RNAse-Inhibitor RNAsin ist und/oder das Antibiotikum aus der Gruppe ausgewählt ist, bestehend aus Proclin-Antibiotika, vorzugsweise Proclin 950 oder Proclin 300, und NaN₃.

3. Verfahren nach Anspruch 1, wobei die Konzentration von Tris-HCl 80 mM bis 120 mM beträgt, die Konzentration von EDTA-2Na 10 mM bis 15 mM beträgt, die Konzentration von Natriumchlorid 0,8 % (w/v) bis 1 % (w/v) beträgt und die Konzentration des RNase-Inhibitors 10 U/ml bis 30 U/ml in der Vorbehandlungslösung beträgt.

4. Verfahren nach einem der Ansprüche 1 bis 3, wobei das Virus ein DNA-Virus oder ein RNA-Virus, vorzugsweise ein RNA-Virus und mehr bevorzugt ein Coronavirus wie 2019-nCoV ist.

5. Vorbehandlungslösung zum Nachweisen einer Nukleinsäure eines Virus, die Vorbehandlungslösung umfassend: Tris-HCl, EDTA-2Na, Natriumchlorid, einen Ribonuklease-Inhibitor (RNase-Inhibitor) und ein Antibiotikum,
wobei die Konzentration von Tris-HCl 10 mM bis 200 mM beträgt, die Konzentration von EDTA-2Na 8 mM bis 50 mM beträgt, die Konzentration von Natriumchlorid 0,5 % (w/v) bis 2 % (w/v) beträgt, die Konzentration des RNase-Inhibitors 2 U/ml bis 800 U/ml beträgt und die Konzentration des Antibiotikums 0,005 % bis 0,05 % beträgt; und
wobei die Vorbehandlungslösung einen pH-Wert von 6,5-8,0 aufweist.

6. Vorbehandlungslösung nach Anspruch 5, wobei der RNAse-Inhibitor RNAsin ist und/oder das Antibiotikum aus der Gruppe ausgewählt ist, bestehend aus Proclin-Antibiotika, vorzugsweise Proclin 950 oder Proclin 300, und NaN₃.

7. Vorbehandlungslösung nach Anspruch 5, wobei die Konzentration von Tris-HCl 80 mM bis 120 mM beträgt, die Konzentration von EDTA-2Na 10 mM bis 15 mM beträgt, die Konzentration von Natriumchlorid 0,8 % (w/v) bis 1 % (w/v) beträgt und die Konzentration des RNase-Inhibitors 10 U/ml bis 30 U/ml beträgt.

8. Vorbehandlungslösung nach 5 bis 7, wobei das Virus ein DNA-Virus oder ein RNA-Virus, vorzugsweise ein RNA-Virus und mehr bevorzugt ein Coronavirus wie 2019-nCoV ist.

9. Verwendung der Vorbehandlungslösung nach einem der Ansprüche 5 bis 7 bei einer Herstellung eines Kits zum Nachweisen eines Virus durch Einsetzen einer Nukleinsäureamplifikation basierend auf dem einstufigen Verfahren.

10. Kit zum Nachweisen einer Nukleinsäure eines Virus basierend auf dem einstufigen Verfahren, das Kit umfassend die Vorbehandlungslösung, die nach einem der Ansprüche 5 bis 7 definiert ist.

11. Kit nach Anspruch 10, wobei das Kit ferner ein Nukleinsäurefreisetzungsmittel und ein qPCR-Amplifikationsreagens umfasst.

## Revendications

1. Méthode de prétraitement pour la détection d'un acide nucléique d'un virus, comprenant : le mélange d'un échantillon stocké dans une solution de prétraitement avec un agent de libération d'acide nucléique et un réactif d'amplification de qPCR, où la solution de prétraitement comprend du Tris-HCl, de l'EDTA-2Na, du chlorure de sodium, un inhibiteur de ribonucléase (RNase), et un antibiotique ;
où la concentration de Tris-HCl est de 10 mM à 200 mM, la concentration d'EDTA-2Na est de 8 mM à 50 mM, la concentration de chlorure de sodium est de 0,5 % (m/v) à 2 % (m/v), la concentration de l'inhibiteur de RNase est de 2 U/mL à 800 U/mL, et la concentration de l'antibiotique est de 0,005 % à 0,05 % dans la solution de prétraitement ; et
où la solution de prétraitement a un pH de 6,5 à 8,0.

2. Méthode selon la revendication 1, dans laquelle l'inhibiteur de l'ARNse est la RNAsin et/ou l'antibiotique est choisi dans le groupe constitué par les antibiotiques Proclin, de préférence Proclin 950 ou Proclin 300, et NaN₃.

3. Méthode selon la revendication 1, dans laquelle la concentration de Tris-HCl est de 80 mM à 120 mM, la concentration d'EDTA-2Na est de 10 mM à 15 mM, la concentration de chlorure de sodium est de 0,8 % (m/v) à 1 % (m/v), et la concentration de l'inhibiteur de RNase est de 10 U/mL à 30 U/mL dans la solution de prétraitement.

4. Méthode selon l'une des revendications 1 à 3, dans laquelle le virus est un virus à ADN ou un virus à ARN, de préférence un virus à ARN, et plus préférentiellement un coronavirus, tel que le 2019-nCoV.

5. Solution de prétraitement pour la détection d'un acide nucléique d'un virus, la solution de prétraitement comprenant : Tris-HCl, EDTA-2Na, chlorure de sodium, un inhibiteur de ribonucléase (RNase) et un antibiotique,
où la concentration de Tris-HCl est de 10 mM à 200 mM, la concentration d'EDTA-2Na est de 8 mM à 50 mM, la concentration de chlorure de sodium est de 0,5 % (m/v) à 2 % (m/v), la concentration de l'inhibiteur de RNase est de 2 U/mL à 800 U/mL, et la concentration de l'antibiotique est de 0,005 % à 0,05 % ; et
où la solution de prétraitement a un pH de 6,5 à 8,0.

6. Solution de prétraitement selon la revendication 5, dans laquelle l'inhibiteur de l'ARNse est la RNAsin et/ou l'antibiotique est choisi dans le groupe constitué par les antibiotiques Proclin, de préférence Proclin 950 ou Proclin 300, et NaN3.

7. Solution de prétraitement selon la revendication 5, dans laquelle la concentration de Tris-HCl est de 80 mM à 120 mM, la concentration d'EDTA-2Na est de 10 mM à 15 mM, la concentration de chlorure de sodium est de 0,8 % (m/v) à 1 % (m/v), et la concentration de l'inhibiteur de RNase est de 10 U/mL à 30 U/mL.

8. Solution de prétraitement selon 5-7, dans laquelle le virus est un virus à ADN ou un virus à ARN, de préférence un virus à ARN, et plus préférentiellement un coronavirus, tel que le

9. Utilisation de la solution de prétraitement selon l'une des revendications 5 à 7 dans la préparation d'un kit de détection d'un virus par amplification d'acide nucléique basé sur la méthode en une étape.

10. Kit de détection d'un acide nucléique d'un virus basé sur la méthode en une étape, le kit comprenant la solution de prétraitement définie selon l'une des revendications 5 à 7.

11. Kit selon la revendication 10, dans lequel le kit comprend en outre un agent de libération d'acide nucléique et un réactif d'amplification de qPCR.
